# FASCICULE DE BREVET EUROPEEN

(11) **EP 1 251 785 B1**
(45) Date de publication et mention de la délivrance du brevet: **01.09.2004**
(21) Numéro de dépôt: 01904052.6
(22) Date de dépôt: 05.02.2001
(51) Int. Cl.: A61B 17/06, A61B 17/04

(54) **AIGUILLE HYPER-ELASTIQUE**
HYPERELASTISCHE NADEL
HYPER-ELASTIC NEEDLE

(30) Priorité: 04.02.2000 FR 0001420
(43) Date de publication de la demande: 30.10.2002
(73) Titulaire: Soprane S.A., 69006 Lyon (FR)
(72) Inventeur: LOUBENS, Thierry, F-69370 Saint-Didier au Mont d'Or (FR); WATRELOT, Antoine, F-69008 Lyon (FR); RIOU, Lionel, F-69003 Lyon (FR)
(74) Mandataire: Schmitt, John
(86) Numéro de dépôt international: PCT/FR2001/000336
(87) Numéro de publication internationale: WO 2001/056478

(56) Documents cités:
- EP-A- 0 529 675
- EP-A- 0 649 633
- WO-A-95/08296
- FR-A- 2 764 500
- US-A- 5 330 441
- US-A- 5 860 991

## Description

La présente invention est relative à des aiguilles chirurgicales fabriquées à partir d'alliages hyper-élastiques qui sont destinées à être utilisées dans les procédures célioscopiques ou endoscopiques.

Le brevet d'invention EP 0529675 du 31 août 1992 au nom de la société ETHICON INC., décrit une aiguille chirurgicale réalisée dans un alliage à mémoire de forme qui a un premier état basse-température, et un second état haute-température.

L'aiguille dans son état basse-température peut être configurée en une forme allongée afin de permettre son passage dans un tube droit.

L'aiguille dans son état haute-température forme un arc prédéterminé, tandis que l'aiguille est adaptée pour être utilisée en tant qu'aiguille chirurgicale.

L'aiguille suivant le brevet EP 0529675 est particulièrement adaptée pour des procédures endoscopiques dans lesquelles des éléments sont conduits dans le site chirurgical par l'intermédiaire d'une canule ou trocart disposant d'un diamètre interne de petite dimension.

L'aiguille décrite dans le brevet EP 0529675 présente certains inconvénients en ce qui concerne l'obligation d'amener le corps de l'aiguille disposée dans le site opératoire, à coté d'une source de chaleur pour qu'elle prenne une configuration courbée suivant un arc prédéterminé.

On constate également que la pointe effilée de l'aiguille risque de se détériorer à l'intérieur de la canule lors de son introduction dans le site opératoire. En effet, le faible diamètre des canules, environ 5 mm, et la forme sensiblement allongée de l'aiguille, conduisent obligatoirement au frottement de sa pointe qui est fragile contre la paroi interne de la canule.

La détérioration de la pointe effilée de l'aiguille est irrémédiable, car le chirurgien ne peut plus l'utiliser, ce qui obligerait ce dernier à introduire une nouvelle aiguille en prenant toutes les précautions possibles d'habilité pour ne pas la détériorer.

L'aiguille chirurgicale suivant la présente invention à pour objet de définir le profil de la pointe effilée pour que cette dernière ne vienne pas se détériorer contre la paroi interne d'une canule ou d'un applicateur spécial, dont le diamètre interne recevant l'aiguille est compris entre 1 et 2 millimètres.

L'aiguille chirurgicale suivant la présente invention est constituée dans un alliage hyper-élastique qui présente, après traitement, deux états distincts permettant, d'une part de contraindre l'aiguille dans une position sensiblement allongée lorsqu'elle est logée dans l'alésage interne d'une canule ou d'un applicateur, et d'autre part lorsqu'elle est extraite de la canule ou de l'applicateur, de pouvoir prendre un profil courbe en arc de cercle du fait de ses propres caractéristiques de superélasticité ou d'hyper-élasticité, et en ce que l'aiguille comporte à l'opposé de la zone de sertissage du fil de suture une pointe effilée pourvue d'un talon incliné qui est disposé à l'intérieur de la courbure de l'aiguille, et qui vient prendre appui contre la paroi interne de l'alésage de la canule ou de l'applicateur pour protéger le profil de la pointe effilée lors des coulissements de ladite aiguille à l'intérieur de l'alésage interne.

L'aiguille chirurgicale suivant la présente invention est constituée dans un alliage hyper-élastique qui comprend environ 54% à 58% de nickel, et environ 42% à 46% de titane.

L'aiguille chirurgicale suivant la présente invention est constituée dans un alliage hyper-élastique qui se compose essentiellement de nickel, de titane et d'une faible quantité de chrome, ou de Fer, ou de Zyrconium, modifiant, soit la température de transformation, soit la dureté dudit alliage.

L'aiguille chirurgicale suivant la présente invention est constituée dans un alliage hyper-élastique qui subit un traitement thermique sous contrainte, dont la température est comprise entre 400°C et 600°C pendant 15 à 60 minutes, suivi d'un refroidissement brutal à l'air ou à l'eau compris entre 0°C et 3°C.

L'aiguille chirurgicale suivant la présente invention comporte une zone de sertissage qui est percée d'un trou borgne interne destiné à recevoir un fil de suture.

L'aiguille chirurgicale suivant la présente invention comporte une zone de sertissage qui peut, dans certains cas, subir un recuit à une température de 500°C suivi d'un refroidissement lent, après mise en forme.

L'aiguille chirurgicale suivant la présente invention comporte un talon qui est incliné d'un angle α qui dépend du diamètre interne de la canule ou de l'applicateur, du diamètre et de la longueur de l'aiguille.

L'aiguille chirurgicale suivant la présente invention présente un profil en section de forme triangulaire dont l'un des sommets du triangle constitue l'arête extérieure de l'arc de cercle de l'aiguille.

L'aiguille chirurgicale suivant la présente invention comporte un talon qui est situé à l'intérieur de la courbure de l'aiguille et à l'opposé de l'arête extérieure du profil triangulaire.

L'aiguille chirurgicale suivant la présente invention comporte une zone de sertissage qui présente un profil en section de forme circulaire.

L'aiguille chirurgicale suivant la présente invention comporte entre la zone de sertissage et la pointe effilée un profil en section de forme sensiblement carrée ou rectangulaire.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue illustrant l'aiguille en alliage hyper-élastique suivant la présente invention.
Figure 2 est une vue montrant en détail la pointe effilée de l'aiguille suivant la présente invention.
Figure 3 est une vue représentant l'aiguille hyper-élastique, contrainte en position allongée, à l'intérieur de l'applicateur de mise en place dans le site opératoire.
Figure 4 est une vue semblable à celle de figure 3, mais illustrant l'aiguille extraite de l'applicateur.
Figure 5 est une vue montrant en détail la position de la pointe effilée de l'aiguille à l'intérieur de l'applicateur.

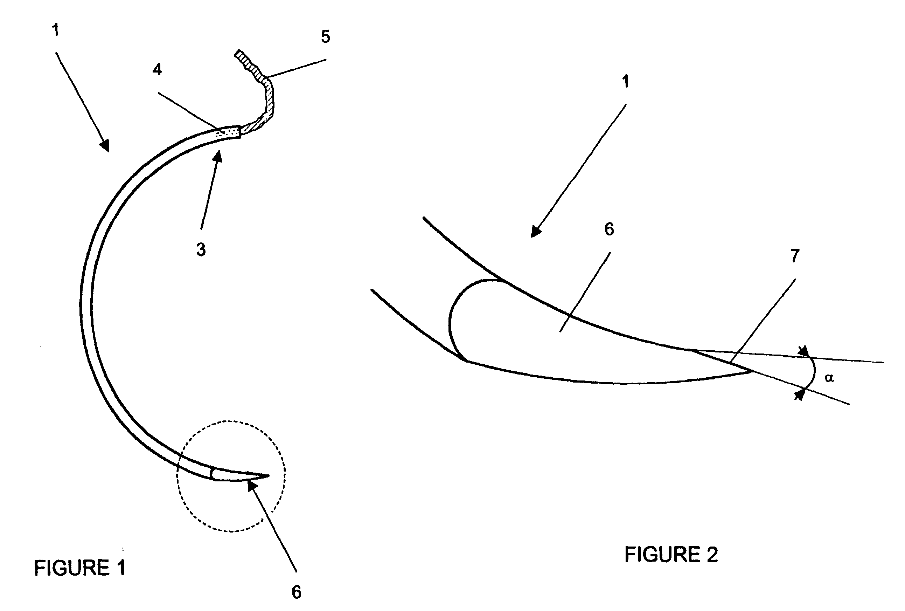

On a montré en figures 1 et 2 une aiguille 1 réalisée dans un alliage hyper-élastique à base de Nickel (Ni), Titane (Ti), qui présente après traitement de l'alliage deux états distincts.

Dans le premier état l'aiguille 1 peut être contrainte dans une position sensiblement allongée pour être disposée à l'intérieur d'une canule ou d'un applicateur 2.

Dans le second état, c'est à dire lorsque la contrainte est supprimée, l'aiguille 1 est conformée suivant un profil en arc de cercle pour son utilisation dans le site chirurgical.

Le passage du premier état au second est inhérent aux caractéristiques de l'alliage qui est traité pour présenter des caractéristiques de superélasticité ou d'hyper-élasticité.

La composition de l'alliage Nickel/Titane varie de 54% à 58% de Nickel, le reste en Titane, c'est à dire de 42% à 46%. Il peut être ajouté d'autres éléments tels que du Chrome (Cr), du Fer (Fe), du Zyrconium (Zr) à de très faibles pourcentages, modifiant, soit la température de transformation, soit la dureté.

La mise en forme de l'aiguille consiste à appliquer un traitement thermique sous contrainte (400°C à 600°C pendant 15 à 60 minutes), suivi d'un refroidissement brutal à l'air ou à l'eau (0°C à 3°C).

L'aiguille 1 comporte une zone de sertissage 3 qui est percée d'un trou borgne interne 4 destiné à recevoir un fil de suture 5 avant sertissage.

La zone de sertissage 3 peut, dans certains cas, subir un recuit (500°C suivi d'un refroidissement lent) après mise en forme. Ce traitement thermique est destiné à supprimer localement l'élasticité de l'alliage dans le but d'améliorer la retenue du fil de suture 5 dans l'aiguille 1.

A l'opposé de la zone de sertissage 3, l'aiguille 1 comporte une extrémité libre qui est conformée suivant le profil d'une pointe effilée 6. La pointe effilée 6 de l'aiguille 1 peut être amincie ou présenter toute autre forme.

La pointe effilée 6 comporte un talon 7 qui est disposé à l'intérieur de la courbure en arc de cercle de l'aiguille 1.

Le talon 7 est incliné d'un angle α par rapport à une tangente horizontale qui dépend du diamètre interne de la canule ou de l'applicateur 2, du diamètre et de la longueur de l'aiguille 1.

Egalement, l'aiguille 1 peut être fabriquée dans toutes les formes connues de la technique chirurgicale. Par exemple, l'aiguille 1 peut disposer en section d'un profil circulaire, carré, rectangulaire ou triangulaire.

il a été préférablement retenu pour la pointe effilée 6 un profil en section de forme triangulaire, dont l'un des sommets du triangle constitue l'arête extérieure de l'arc de cercle de l'aiguille 1. Dans ce cas, le talon 7 est situé à l'intérieur de la courbure de l'aiguille 1 et à l'opposé de l'arête extérieure du profil triangulaire.

L'aiguille 1 présente au niveau de la zone de sertissage 3 un profil en section de forme circulaire, tandis que le reste du corps de l'aiguille qui est compris entre la pointe effilée 6, et ladite zone de sertissage 3 présente un profil en section de forme sensiblement carrée ou rectangulaire.

Le profil en section de forme sensiblement carrée ou rectangulaire du corps de l'aiguille 1 permet au chirurgien de mieux saisir cette dernière dans une pince ou porte aiguille.

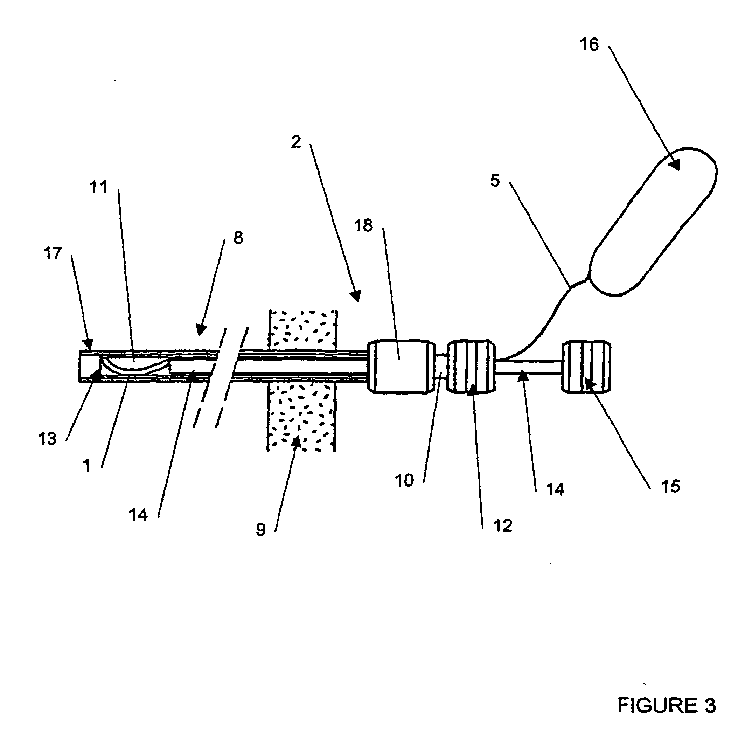

On a représenté en figure 3 l'applicateur 2 permettant la mise en place de l'aiguille 1 solidaire de son fil de suture 5 dans le site chirurgical par l'intermédiaire d'un trocart 8 qui a été préalablement placé au travers de la paroi 9 d'un patient.

Cet applicateur a été décrit et protégé dans un brevet français n° 97 07681 (publié FR-A-2 764 500) appartenant au demandeur.

L'applicateur 2 comprend un manchon cylindrique 10 percé sur toute sa longueur, et parallèlement à son axe longitudinal, d'un alésage 11 dans lequel sont logés l'aiguille 1 et son fil de suture 5.

Le manchon 10 comporte à l'une de ses extrémités une tête cylindrique 12 qui est prévue d'un diamètre supérieur à celui du reste du corps dudit manchon.

L'aiguille 1 est disposée dans l'alésage 11 du manchon 10 de manière que sa pointe effilée 6 soit dirigée en direction de l'extrémité libre 13 et opposée à celle portant la tête 12.

L'applicateur 2 comprend une tige 14 qui est solidaire. à l'une de ses extrémités d'une butée cylindrique 15

La tige 14 est introduite dans le manchon 10 de manière à venir en appui contre l'aiguille 1. La tige 14 traverse l'alésage 11, de manière que le fil de suture 5 soit disposé entre ladite tige et la paroi dudit alésage.

Des moyens de retenue 16 permettent de maintenir la tige 14 par rapport au manchon 10, de manière que l'extrémité libre de la tige 14 opposée à la butée 15 soit toujours en appuie contre l'aiguille 1.

L'applicateur 2 est disposé à l'intérieur du trocart 8 qui a été préalablement placé au travers de la paroi 9 d'un patient.

Le trocart est constitué d'un tube 17 solidaire à l'une de ses extrémités d'une jupe circulaire 18 servant de butée à l'applicateur lors de la mise en place de l'aiguille 1 dans le site chirurgical comme on le verra mieux plus loin.

Le tube 17 du trocart 8 est prévu pour recevoir le manchon 10 de l'applicateur 2, tandis que la tête 12 vient en appuie contre la jupe circulaire 18.

Le chirurgien procède ensuite au retrait des moyens de retenue 16 pour libérer la tige 14 par rapport au manchon 10.

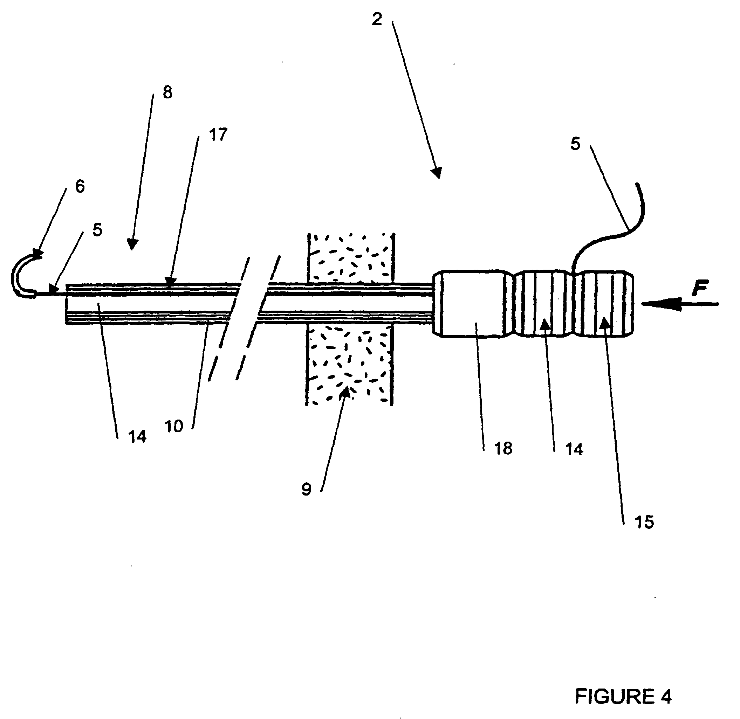

En figure 4 on a montré l'extraction de l'aiguille 1 du manchon 10 de l'applicateur 2.

En effet, il suffit au chirurgien d'appuyer, suivant la flèche F, sur la butée 15 de la tige 14, pour pousser, par l'intermédiaire de ladite tige 14, l'aiguille 1 à l'extérieur du manchon 10. Le déplacement de la tige 14 est limité dans sa course jusqu'à ce que la butée 15 soit en contact avec la tête 12 du manchon 10.

Lorsque l'aiguille 1 est extraite du manchon 10 de l'applicateur, cette dernière, du fait de son élasticité, prend sa forme en arc de cercle, permettant au chirurgien de réaliser les points de sutures.

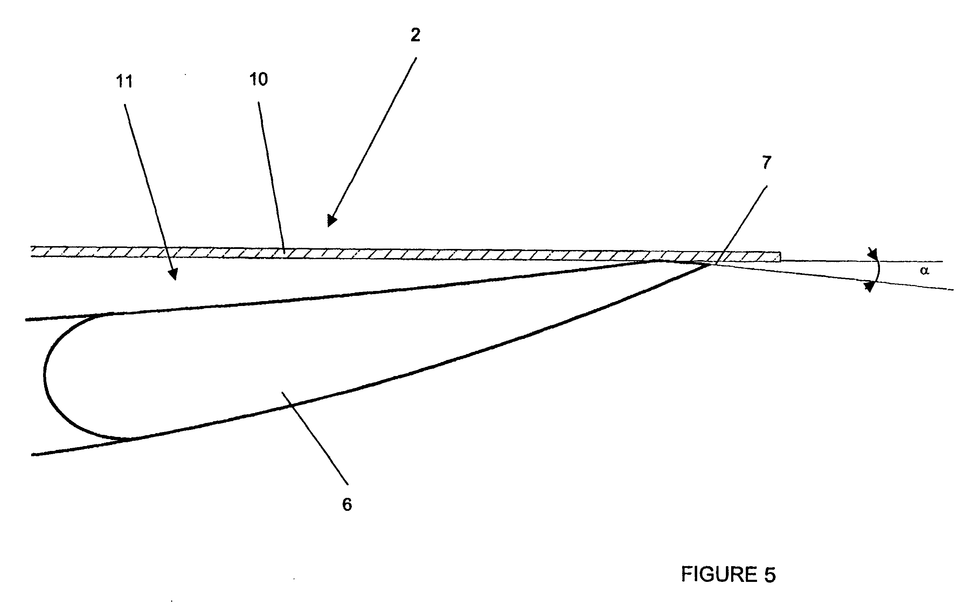

En figure 5 on a montré la position de la pointe effilée 6 de l'aiguille 1 à l'intérieur de l'alésage 11 du manchon 10 de l'applicateur 2.

On note que le talon 7 permet de protéger la pointe effilée 6 de l'aiguille lors de ses déplacements ou coulissements à l'intérieur de l'alésage 11 du manchon 10.

En effet, seul le talon 7, et plus particulièrement le point de raccordement avec la partie courbe de l'aiguille 1, est en contact avec la paroi interne de l'alésage 11 du manchon 10 protégeant ainsi la pointe effilée 6 contre toute détérioration par frottement.

Il va de soi que l'applicateur 2 peut être remplacé par une canule ou un trocart connu en soi pour la mise en place de l'aiguille 1 à l'intérieur du site opératoire sans pour autant changer l'objet de la présente invention.

## Revendications

1. Aiguille chirurgicale comportant une zone de sertissage (3) pour la fixation d'un fil de suture (5), ladite aiguille étant constituée dans un alliage hyper-élastique qui présente, après traitement, deux états distincts permettant, d'une part de contraindre l'aiguille dans une position sensiblement allongée lorsqu'elle est logée dans l'alésage interne (11) d'une canule ou d'un applicateur (2), et d'autre part lorsqu'elle est extraite de la canule ou de l'applicateur de pouvoir prendre un profil courbe en arc de cercle du fait de ses propres caractéristiques de superélasticité ou d'hyper-étasticité, et comporte à l'opposé de la zone de sertissage (3) une pointe effilée (6) **caractérisée en ce que** l'aiguille est pourvue d'un talon incliné (7) qui est disposé à l'intérieur de la courbure de l'aiguille (1) et qui vient prendre appui contre la paroi interne de l'alésage (11) de la canule ou de l'applicateur (2) pour protéger le profil de la pointe effilée (6) lors des coulissements de ladite aiguille à l'intérieur de l'alésage interne (11).

2. Aiguille chirurgicale suivant la revendication 1, **caractérisée en ce que** l'alliage hyper-élastique comprend environ 54% à 58% de nickel et environ 42% à 46% de titane.

3. Aiguille chirurgicale suivant la revendication 2, **caractérisée en ce que** l'alliage hyper-élastique se compose essentiellement de nickel, de titane et d'une faible quantité de chrome, ou de Fer, ou de Zyrconium, modifiant, soit la température de transformation, soit la dureté dudit alliage.

4. Aiguille chirurgicale suivant la revendication 1, **caractérisée en ce que** l'alliage hyper-élastique constituant l'aiguille (1) a subit un traitement thermique sous contrainte dont la température est comprise entre 400°C et 600°C pendant 15 à 60 minutes, suivi d'un refroidissement brutal à l'air ou à l'eau compris entre 0°C et 3°C.

5. Aiguille chirurgicale suivant la revendication 1, **caractérisée en ce qu'**elle comporte une zone de sertissage (3) qui est percée d'un trou borgne interne (4) destiné à recevoir un fil de suture (5).

6. Aiguille chirurgicale suivant la revendication 5, **caractérisée en ce que** la zone de sertissage (3) a subit un recuit à une température de 500°C suivi d'un refroidissement lent, après mise en forme.

7. Aiguille chirurgicale suivant la revendication 1, **caractérisée en ce que** le talon (7) est incliné d'un angle α qui dépend du diamètre interne de la canule ou de l'applicateur (2), du diamètre et de la longueur de l'aiguille (1).

8. Aiguille chirurgicale suivant la revendication 1, **caractérisée en ce qu'**elle présente au niveau de la pointe effilée (6) un profil en section de forme triangulaire, dont l'un des sommets du triangle constitue l'arête extérieure de l'arc de cercle de l'aiguille (1).

9. Aiguille chirurgicale suivant la revendication 8, **caractérisée en ce que** ledit talon (7) est situé à l'intérieur de la courbure de l'aiguille (1) et à l'opposé de l'arête extérieure du profil triangulaire.

10. Aiguille chirurgicale suivant la revendication 1, **caractérisée en ce qu'**elle présente au niveau de la zone de sertissage (3) un profil en section de forme circulaire.

11. Aiguille chirurgicale suivant la revendication 1, **caractérisée en ce qu'**elle présente entre la zone de sertissage (3) et la pointe effilée (6) un profil en section de forme sensiblement carrée ou rectangulaire.

## Patentansprüche

1. Chirurgische Nadel, die eine Crimpzone (3) zur Befestigung eines Nähfadens (5) umfaßt, wobei die Nadel aus einer hyperelastischen Legierung besteht, die, nach Behandlung, zwei unterschiedliche Zustände aufweist, welche es einerseits erlauben, die Nadel in eine annähernd ausgestreckte Position zu zwingen, wenn sie in der inneren Bohrung (11) einer Kanüle oder eines Applikators (2) beherbergt ist und andererseits, wenn sie aus der Kanüle oder dem Applikator gezogen wird, ein gebogenes Profil in Form eines Kreisbogens annehmen zu können aufgrund ihrer besonderen Eigenschaften der Superelastizität oder Hyperelastizität und die gegenüber der Crimpzone (3) eine sich verjüngende Spitze (6) umfaßt, die mit einem schrägen Absatz (7) versehen ist, der sich innerhalb der Krümmung der Nadel (1) befindet und der sich an die innere Wandung der Bohrung (11) der Kanüle oder des Applikators (2) anlegt, um das Profil der sich verjüngenden Spitze (6) beim Gleiten der Nadel im Innern der inneren Bohrung (11) zu schützen.

2. Chirurgische Nadel nach Anspruch 1, **dadurch gekennzeichnet, daß** die hyperelastische Legierung zwischen ungefähr 54 % und 58 % Nickel und ungefähr 42 % bis 46 % Titan umfaßt.

3. Chirurgische Nadel nach Anspruch 2, **dadurch gekennzeichnet, daß** sich die hyperelastische Legierung im wesentlichen aus Nickel, Titan und einer geringen Menge an Chrom, Eisen oder Zirkonium zusammensetzt, wobei entweder die Transformationstemperatur oder die Härte der Legierung verändert wird.

4. Chirurgische Nadel nach Anspruch 1, **dadurch gekennzeichnet, daß** die die Nadel (1) bildende hyperelastische Legierung einer Hitzebehandlung unter Streß, wobei die Temperatur während 15 bis 60 Minuten zwischen 400°C und 600°C lag, gefolgt von einer Schockkühlung an der Luft oder im Wasser bei zwischen 0 °C und 3 °C unterzogen wurde.

5. Chirurgische Nadel nach Anspruch 1, **dadurch gekennzeichnet, daß** sie eine Crimpzone (3) umfaßt, die von einer internen Sackbohrung (4) durchbohrt ist, welche zur Aufnahme eines Nähfadens (5) gedacht ist.

6. Chirurgische Nadel nach Anspruch 5, **dadurch gekennzeichnet, daß** die Crimpzone (3) einem Ausglühen bei einer Temperatur von 500 °C gefolgt von einer langsamen Abkühlung, nach der Formung, unterzogen wurde.

7. Chirurgische Nadel nach Anspruch 1, **dadurch gekennzeichnet, daß** der Absatz (7) in einem Winkel α geneigt ist, der vom inneren Durchmesser der Kanüle oder des Applikators (2), des Durchmessers und der Länge der Nadel (1) abhängt.

8. Chirurgische Nadel nach Anspruch 1, **dadurch gekennzeichnet, daß** sie an der sich verjüngenden Spitze (6) ein Schnittprofil in Form eines Dreiecks aufweist, wobei eine Spitze des Dreiecks die Außenkante des Kreisbogens der Nadel (1) bildet.

9. Chirurgische Nadel nach Anspruch 8, **dadurch gekennzeichnet, daß** sich der Absatz (7) innerhalb der Krümmung der Nadel (1) und gegenüber der Außenkante des dreieckigen Profils befindet.

10. Chirurgische Nadel nach Anspruch 1, **dadurch gekennzeichnet, daß** sie an der Crimpzone (3) ein Schnittprofil in zirkulärer Form aufweist.

11. Chirurgische Nadel nach Anspruch 1, **dadurch gekennzeichnet, daß** sie zwischen der Crimpzone (3) und der sich verjüngenden Spitze (6) ein Schnittprofil von annähernd quadratischer oder rechteckiger Form aufweist.

## Claims

1. Surgical needle comprising a crimping zone (3) for securing a suture (5), said needle being made of a hyperelastic alloy which, after treatment, has two distinct states making it possible, on the one hand, to force the needle into a roughly elongate position when it is housed in the internal bore (11) of a cannula or of an applicator (2) and, on the other hand, when it is extracted from the cannula or from the applicator, to adopt a curved profile in the shape of an arc of a circle because of its own characteristics of superelasticity or hyperelasticity, and comprising, at the opposite end to the crimping zone (3), a tapered point (6), **characterized in that** the needle is provided with an inclined heel (7) which is arranged inside the curvature of the needle (1) and which bears against the interior wall of the bore (11) of the cannula or of the applicator (2) to protect the profile of the tapered point (6) as said needle slides inside the internal bore (11).

2. Surgical needle according to Claim 1, **characterized in that** the hyperelastic alloy contains about 54% to 58% nickel and about 42% to 46% titanium.

3. Surgical needle according to Claim 2, **characterized in that** the hyperelastic alloy is made up essentially of nickel, titanium and a small amount of chromium, or iron, or zirconium, modifying either the transformation temperature or the hardness of said alloy.

4. Surgical needle according to Claim 1, **characterized in that** the hyperelastic alloy of which the needle (1) is made has undergone a heat treatment under stress, the temperature of which is between 400°C and 600°C for 15 to 60 minutes, followed by a sudden quenching in air or water at between 0°C and 3°C.

5. Surgical needle according to Claim 1, **characterized in that** it comprises a crimping zone (3) which is pierced with an internal blind hole (4) intended to house a suture (5).

6. Surgical needle according to Claim 5, **characterized in that** the crimping zone (3) has undergone annealing at a temperature of 500°C, followed by slow cooling, after shaping.

7. Surgical needle according to Claim 1, **characterized in that** the heel (7) is inclined by an angle α which depends on the internal diameter of the cannula or of the applicator (2), on the diameter and on the length of the needle (1).

8. Surgical needle according to Claim 1, **characterized in that**, at the tapered point (6), it has a cross-sectional profile of triangular shape, one of the vertices of which triangle constitutes the outer edge of the arc of a circle of the needle (1).

9. Surgical needle according to Claim 8, **characterized in that** said heel (7) lies on the inside of the curvature of the needle (1) and on the opposite side to the outer edge of the triangular profile.

10. Surgical needle according to Claim 1, **characterized in that**, in the crimping zone (3), it has a cross-sectional profile of circular shape.

11. Surgical needle according to Claim 1, **characterized in that**, between the crimping zone (3) and the tapered point (6), it has a cross-sectional profile of roughly square or rectangular shape.
